# EUROPEAN PATENT APPLICATION

(11) **EP 1 234 873 A1**
(43) Date of publication of application: **28.08.2002**
(21) Application number: 01104062.3
(22) Date of filing: 20.02.2001
(51) Int. Cl.: C12N 1/20, C12Q 1/02, C12M 1/34, C12Q 1/00, C07K 1/04

(54) **Indicator medium for the detection of contaminants in gene libraries**

(71) Applicant: Deutsches Ressourcenzentrum für Genomforschung GmbH, 14059 Berlin (DE)
(72) Inventor: Kirby, Susan F., Bishps Stortford Herts CM23 3SD (GB); Mielke, Martin, Prof., 13353 Berlin (DE); Koller, Barbara, Dr., 14195 Berlin (DE); Peters, Marika, 13595 Berlin (DE); Schneider, Dirk, 13407 Berlin (DE)
(74) Representative: VOSSIUS & PARTNER

(57) **Abstract**

The present invention is a growth medium comprising a compound or a mixture of compounds wherein said growth medium restricts growth of microorganisms having a defect in at least one factor which is required to metabolize said compound or mixture of compounds and wherein said growth medium promotes growth of microorganisms lacking said defect.

Further, the present invention relates to the use of the growth medium for the detection of microorganisms contaminating gene libraries.

Additionally, the present invention relates to the method for the detection of contaminating microganisms in gene libraries which consist of microorganisms having a defect in at least one factor which is required to matabolize said compound or mixture of compounds comprising the medium.

Furthermore, the present invention relates to the steps of (a) growing said gene libraries on the growth medium of the invention and (b) identifying said contaminating microorganisms by visualizing their metabolic activities.

Finally, the present invention relates to a kit suitable for carrying out the method of the present invention.

## Description

The present invention relates to a growth medium comprising a compound or a mixture of compounds, wherein said growth medium restricts growth of microorganisms having a defect in at least one factor which is required to metabolize said compound or mixture of compounds and wherein said growth medium promotes growth of microorganisms lacking said defect. Further, the present invention relates to the use of the growth medium of the present invention for the detection of microorganisms contaminating a gene library. Additionally, the present invention relates to a method for the detection of contaminating microorganisms in a gene library consisting of microorganisms having a defect in at least one factor which is required to metabolize said compound or mixture of compounds comprising the steps of (a) growing said gene library on the growth medium of the invention and (b) identifying said contaminating microorganism by visualizing its metabolic activity. Finally, the present invention relates to a kit suitable for carrying out the method of the present invention.

The use of gene libraries is of increasing importance in all parts of biotechnology. This complex biological material is usually stored and made available by Gene Bank Resource Centers throughout the world. Thus, the Resource Center of the German Human Genome Project is a large gene bank which maintains about 300 gene libraries comprising more than 20 million individual E. coli based clones. The clones are arrayed in 384 well microtitre plates in liquid media and stored at -80°C. These libraries are both created in or supplied to the Resource Center, from where they are distributed as individual clones in agar stabs or as colony filters to a large number of research institutes.

An important pre-requisite for maintaining and distributing large collections of gene libraries is the microbial purity of the clones. Antibiotic resistant contaminating micro-organisms have been found in many E. coli based gene libraries. One of the largest library collections distributed by the German Resource Center is supplied by the IMAGE consortium (Ref. 1). As a distributor of this resource the present inventors have reported over 100,000 contaminants to the consortium. Contamination may spread in the microtitre plates during repeated cycles of replication and most importantly they can outgrow and destroy large parts of a library. As the Resource Center acts as a distributor for the clones, contaminants can also infect and spread into other research libraries.

There have been previous efforts in the art aiming at an easy recognition and early eradication of contaminants. These efforts relied on a visual or metabolic distinction between contaminating microorganisms and microorganisms carrying the recombinant vectors/plasmids and thus forming, in the sense of the present invention, a gene library. As a rule, these prior art media were designed to suppress growth of the contaminants. For example, in order to keep a high level of microbiological purity in an E. coli based library, the present inventors searched for an indicator medium which was capable of distinguishing between transformed E. coli clones forming a gene library and contaminants, including wild type E. coli. The first step was to find a commercially available medium for this purpose. Commercial media are usually designed to select for certain types of microorganisms. Therefore, they often contain inhibitors for other organisms and therefore these organisms cannot be detected. In collaboration with the company Merck, Darmstadt, Germany, a modification of their Chromocult medium Chromocult Pure Coli Clone Agar (PCC) (Ref. 2) was developed. This medium is free of inhibitors and it can indicate and confirm the presence of the E. coli clones forming said gene library. Substantial overgrowth by a contaminant or the complete loss of a library clone can be visualised. However, incipient growth of a contamination is not detected. Undetected low-level contaminants may result in the destruction of the clone during the next round of replication. Furthermore, this medium cannot differentiate between an E. coli clone belonging to the library and wild type E. coli.

Prior art media consequently do not easily or early enough distinguish between microorganisms forming a gene library and contaminating microorganisms. Therefore, at present there is a risk that available gene libraries are destroyed by contaminants, since these may not be timely detected to prevent destruction of significant portions of or complete libraries. Due to distribution of contaminated gene libraries all over the world that risk is currently increasing and may also affect other as yet not contaminated gene libraries which may be locally stored together with the contaminated gene libraries.

Thus, the technical problem underlying the present invention was to provide means and methods for the easy and/or early detection of microbial contaminants in gene-libraries.

The solution to said technical problem is provided by the embodiments characterized in the claims.

Accordingly, the present invention relates to a growth medium comprising a compound or a mixture of compounds, wherein said growth medium restricts growth of microorganisms having a defect in at least one factor which is required to metabolize said compound or mixture of compounds and wherein said growth medium promotes growth of microorganisms lacking said defect.

The term "growth medium" as used herein refers to both aqueous solutions as well as solid/solidified media.

The term "microorganisms having a defect in at least one factor which is required to metabolize said compound or mixture of compounds" means, in accordance with the present invention, the following. Microorganisms rely for the generation of energy (and thus for growth) on the metabolization of external substrates, as is well known in the art. The catabolism of such external substrates is strictly regulated by the microorganisms and involves enzymes which are required in a metabolic pathway and proteins which are involved in transcriptional, translational and/or post-translational regulation of said enzymes. Said enzymes and other proteins are included in the term "factor" as used herein. Once such a factor is not produced or produced in suboptimal amounts by the microorganisms, the metabolism of the substrate is impaired or abolished. It is self-evident that metabolization of a substrate is usually effected by a cascade of enzymatic events in which different enzymes are involved. Therefore, a factor which is required to metabolize a substrate may relate to different enzymes (or different other proteins) that act along the pathway of metabolizing the substrate. Similarly, the invention comprises situations wherein more than one factor along such a pathway is defect. A substrate which cannot/can no longer be metabolized by said microorganism is contained in or added to the growth medium of the invention and also termed a "compound". As stated above, the growth medium may contain a mixture of compounds that cannot be metabolized by the microorganisms due to the absence of at least one functional factor required for the metabolization of the mixture of compounds. This embodiment envisages situations where the microorganism has various, such as two, three, four, or more defects in factors involved in the metabolization of different, such as two, three or more compounds. Again, basically two different situations may be envisaged. The first is that the microorganism displays one defective factor in each of the pathways. Alternatively, more than one factor may be defective at least in some and possible each of the pathways for which the compounds contained in or added to said growth medium form a substrate.

The compounds which are necessary for the growth of microorganisms may be substrates such as carbohydrates, amino acids or derivatives thereof, or inorganic substances.

The term "gene libraries" as used herein means a variety of recombinant plasmids, cosmids or other vectors containing a cDNA, genomic or other gene bank. Alternatively, said term refers to a collection of microorganisms carrying such a gene bank, usually as the result of transformation/transfection process. Said gene libraries may preferably be spotted on a solid support and/or could be maintained in microtitre plates.

In accordance with the present invention, a variety of microorganisms commonly used in the generation of gene libraries and exemplified by E. coli strains were tested for their capacity to metabolize a variety of different compounds. These compounds which were not metabolized by the test library strains were checked in an extensive database search to identify metabolic activities highly prevalent in potential contaminants. Further in accordance with the invention, a number of compounds could be identified that are metabolized by these contaminants but not by E. coli based gene libraries. These results obtained for E. coli and the means to obtain such results can, without further ado, be transferred to gene libraries not based on E. coli such as yeast gene libraries. Accordingly, microorganisms such as yeast (e.g. S. cerevisiae or P. pastoris) or bacteria (e.g. E.coli) having a defect as referred to above are, in accordance with the invention, microorganisms that are either useful, used or suitable for, or in transfected/transformed form, constitute gene libraries. Consequently, microorganisms lacking a defect as described above are also referred to as contaminants.

The growth medium of the present invention is designed to make use of the different metabolic activities of library strains and contaminants. It contains compounds that are metabolized by the contaminants but not by the library strains and thus promotes growth of the contaminants. The growth medium of the invention is further designed to contain only components that allow a retarded growth of the library strains when compared to the growth of the contaminants. The design of the growth medium of the present invention makes use of the opposite approach as compared to the above discussed prior art media. Namely, the growth medium of the present invention promotes rather than restricts growth/proliferation of the contaminants.

As a consequence of the medium design, the growth medium of the present invention promotes growth of said contaminants and at the same time restricts growth of microorganisms constituting said gene libraries. It thus forms the basis for an efficient and reliable detection of said contaminants. Due to its advantageous properties, it allows even the detection of low-level contaminants which may not yet have become an evident problem for the purity of a gene library but which will become a risk within a few more additional life cycles. Advantageously, the above described early detection of contaminants based on the growth medium of the present invention allows to recover microorganisms constituting a gene library and dissecting them from contaminants.

The detection of the contaminants can be effected by a variety of means. Due to the growth promotion of the contaminants, detection within the gene library may be performed visually over time. If the gene library is stored in 384 microtiter plates, for example, visual inspection will lead to the detection of fast growing contaminating colonies. The detection process is further simplified if the growth medium additionally contains a chromogenic substrate or chromogen which changes color upon the metabolic activity of the contaminants. For example, the metabolic activities of the contaminants may result in a pH change of the growth medium which can be monitored with Phenol Red. In another embodiment, the contaminants may be detected due to the metabolism of the chromogenic substrate ONPG by the contaminants.

The separation of contaminants and library clones may be achieved either by discarding the contaminants from the library or by isolation of the library clones from the contaminated gene library. Both methods may be assisted by automation, e.g. by way of picking/spotting robots. Picking may further be assisted by automated visual means such as a CCD camera. In one embodiment, valuable clones are rescued by streaking out contaminated libraries on an agar plate containing the growth medium of the invention and re-isolating the pure, valuable clones.

The growth medium of the present invention may find application in a variety of situations for rescuing valuable libraries or clones therefrom: Original cDNA libraries are highly redundant, and single contaminated clones may be removed without significantly changing the quality of the library. However, as more sequences of genes become available, sets of non-redundant clones each representing a unique gene are being made by re-arraying of the redundant gene banks. For the preparation of these "unigene sets" detection and exclusion of contamination are even more important as for the maintenance of redundant libraries. The growth medium of the invention can also be used to rescue valuable single clones of non-redundant gene sets. The contaminated clone can be streaked out on an agar plate with the growth medium of the invention and a clean colony can be recovered and used to replace the contaminated colony in the unigene set.

In summary, the growth medium of the invention can be particularly well employed for the following purposes:
a) quality control of large gene libraries during production, storage, application and reproduction processes.
b) quality control of the products derived from large gene libraries, e.g. hybridisation filter, stab cultures of single clones, DNA microarrays.
c) guarantee of absence of contamination during the production maintenance and application of sets of unique genes.
d) rescue of valuable single clones in sets of unique genes.

In a preferred embodiment of the growth medium of the present invention said microorganism lacking said defect is a bacterium.
Bacteria in the sense of this preferred embodiment which are often encountered as contaminants of gene libraries are exemplified by wildtype E. coli, Pseudomonas and Bacillus subtilis.

In a further preferred embodiment of the growth medium of the present invention said microorganism having said defect is a bacterium.
Bacteria having such a defect and which are often chosen as a source for the establishment of gene libraries are exemplified by E. coli.

Bacterial strains exhibiting defects in metabolism are frequently used in gene libraries. Such bacterial strains comprising mutations in their genomes are described in the prior art and widely available. The resulting differences in the genetic background of bacterial strains exhibiting defects in metabolism and corresponding wild type bacterial strains which lack those defects can be taken into consideration for the composition of a growth medium of the present invention beyond the specific teaching of this specification as disclosed in the appended examples.

In a particularly preferred embodiment said bacterium is E.coli.

E. coli strains are widely distributed as sources/carriers of gene libraries, as is well known in the art. The present invention is therefore expected to find particular application in the protection of E. coli based libraries.

In a furthermore preferred embodiment said E.coli comprises a defective lac-operon and lacks the ability to metabolize saccharose, inosite and sodium thiosulfate.

E. coli strains which comprise a defective lac-operon are particularly well-suited and are very well-known in the art as being useful for gene libraries. In accordance with the present invention growth of said E. coli strains comprising a defective lac-operon may be restricted by a growth medium of the invention, lacking substrates which can be metabolized even in the absence of a functional lac-operon. The components of said growth medium and can therefore only be metabolized by contaminants, such as bacterial strains having a functional lac-operon. Said substrates may be selected from the group consisting of: saccharose, inosite and sodium thiosulfate. E. coli strains comprising a defective lac-operon as used herein may be for example DH10B, DH12S, XL-1 Blue, XL-2Blue and DH5alpha.

In accordance with the foregoing, in a most preferred embodiment of the growth medium of the present invention said E.coli is selected from the group consisting of DH10B, DH125, XL-1Blue, XL-2Blue and DH5α.

In a preferred embodiment of the growth medium of the present invention said compound is or said mixture of compounds comprises a carbohydrate.
To identify suitable substrates and to develop the growth medium presented in this invention, the ability of the test strains to metabolise 49 different sugars and 11 other substrates was tested. The 28 substrates were not metabolised by the test strains. These substrates were used in an extensive database search to identify metabolic activities highly prevalent in potential contaminants. The analysis resulted in the identification of Lactose/ONPG, Saccharose, Inosit, Fe(III)-citrate and Sodiumthiosulfate as the most suitable substrates for the identification of metabolic activities of contaminants. In the growth medium these six substrates are dissolved in a suitable broth. Metabolic activities of contaminants result either in a pH change, which is monitored with Phenol Red, or in the metabolism of the chromogenic substrate ONPG.
However, contamination by ubiquitous non-fermenters such as Pseudomonas aeruginosa, Spheromonas paucimobilis, Stenotrophomonas maltophilia, and some strains of Acinetobacter spp. do not result in a colour change, because of the lack of sugar metabolism. These bacteria can be recognized by the significantly different morphology of the colonies and a red colour. Preferably, for improved identification of such contaminants Arbutin is included in the medium as an additional substrate.

In light of the foregoing, in a more preferred embodiment of the growth medium of the present invention said carbohydrate is selected from the group consisting of: erythritol, D-arabinose, L-xylose, adonitol, β-methyl-xyloside, L-sorbose, dulcitol, inositol, alpha-methyl-D-mannoside, alpha-methyl-D-glucoside, lactose, saccharose, inuline, melezitose, D-raffinose, D-turanose, D-lycose, D-tagatose, D-fucose, D-arabitol, L-arabitol, amygdaline, arbutin, esculine, amidon, and ONPG (=ortho-nitro-phenyl-galactoside).

Also, in another preferred embodiment of the growth medium of the present invention said compound is or said mixture of compounds comprises an amino acid or derivative thereof.

Moreover, in a more preferred embodiment of the growth medium of the present invention said amino acid or derivative thereof is selected from the group consisting of: arginine, ornithine, tryptophane, and urea.

In a further preferred embodiment of the growth medium of the present invention said compound is or said mixture comprises an inorganic compound.

More preferably, said inorganic compound is selected from the group consisting of: sodium citrate, sodium thiosulfate, and sodium pyruvate.

In a further preferred embodiment of the growth medium of the present invention said compound is or said mixture of compounds comprises a complex substance.

More preferably, said complex substance is Kohns gelatine.

Also preferred in accordance with the invention is a growth medium, wherein said mixture comprises at least saccharose, inosite, iron (III) citrate, sodium thiosulfate and ONPG.

In a most preferred embodiment the growth medium of the invention has the composition as indicated in Example 2.

In a more preferred embodiment said metabolization allows visual detection of microbial contaminants of gene libraries.
"Visual detection" allowed by said metabolization as used herein means that the growth of said contaminants of gene libraries can be detected visually due to the metabolizing activities of said contaminants. Visual detection may simply be effected by assessing faster growth and observing larger colonies of contaminants as compared to gene library clones. Alternatively, visual detection may rely on chemical modifications of, e.g., substrates during the metabolization procedures as has been mentioned herein above. Said modifications may also comprise an altered composition of the micro-environment comprising the pH of the growth medium. It is well-known that chromogens may change the color of a solution due to changes in the pH. Such a chromogen may be, e.g., Phenol Red which changes the color of a Phenol Red containing solution from red to yellow or vise versa dependent on the respective pH of said solution. Notwithstanding, the term "chromogens" in accordance with the invention comprises all substances which can be visualized. Usually, said chromogens comprise a chromophore. In accordance with the present invention said chromphore will become activated due to the metabolic activity of the microbial contaminants. Moreover, also suitable for visual analysis are chromogenic substrates. Chromogenic substrates, due to the metabolization procedure, become activated resulting in a detectable color change or the appearance of a detectable visual signal. A chromogenic substrate used in the growth medium in accordance with the present invention is ONPG. As discussed elsewhere in this specification, visual detection may be assisted by automation.

In accordance with the above, in a more preferred embodiment of the growth medium of the invention said visual analysis is based on pH-dependent color change of a chromogen.

In a most preferred embodiment of the growth medium of the invention said chromogen is Phenol Red.

Further in accordance with the above, in another more preferred embodiment of the growth medium of the invention said visual analysis is based on the metabolism of a chromogenic substrate.

In a most preferred embodiment of the growth medium of the invention said chromogenic substrate is ONPG.

In a furthermore preferred embodiment of the growth medium of the invention said visual analysis is based on the metabolism of sodium thiosulfate.

Most preferably, said metabolism of sodium thiosulfate is visualized by reaction with iron-III-citrate.

Catalyzed by the thiosulfate metabolism of contaminants, iron-III-citrate may be obtained which changes the color of a solution comprising it such as the growth medium of the invention. Advantageously, said color change can be detected by eye and does not need to apply further technical devices.

In a most preferred embodiment, the growth medium of the present invention further comprises Arbutin.

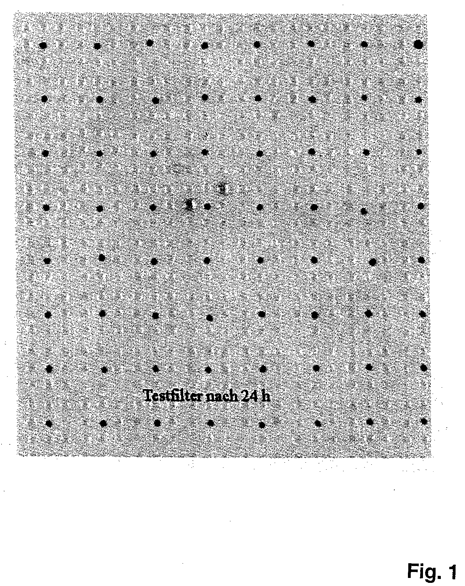

Contamination by ubiquitous non-fermenters such as Pseudomonas aeruginosa, Spheromonas paucimobilis, Stenotrophomonas maltophilia, and some strains of Acinetobacter spp. may not result in a color change when grown on the growth medium of the invention, because of the lack of sugar metabolism. In most cases these bacteria may be recognized by the significantly different morphology of the colonies and a red color (Figure 1B). For improved identification of such contaminants Arbutin may be included in the medium as an additional substrate.

In a further preferred embodiment of the growth medium of the invention said microorganism lacking said defect is yeast.
By adapting the teachings of the present invention to yeast contaminants, the growth medium of the present invention is expected to successfully separate such yeast contaminants from gene libraries.

Also, in a preferred embodiment of the growth medium of the invention said microorganism having said defect is yeast.
Yeast is, besides bacteria, the type of organism most commonly used in gene libraries. Therefore, the growth medium of the invention can be adapted to and employed in the protection of yeast based gene libraries from contaminants.

The definitions of the terms with respect to the growth medium of the present invention discussed herein before can be applied mutatis mutandis to the following embodiments relating to uses of the growth medium of the present invention and to methods comprising the growth medium of the present invention.

The present invention also relates to the use of the growth medium of the invention for the detection of contaminating microorganisms in gene libraries.

In a more preferred embodiment said contaminants are bacteria.

Furthermore preferably, said gene library is based on bacteria.

In a most preferred embodiment of the use of the present invention said bacteria are E. coli.

In a further most preferred embodiment of the use of the present invention said E. coli comprise a defective lac-operon and lack the ability to metabolize saccharose, inosite and sodium thiosulfate.

In another further most preferred embodiment of the use of the present invention said E. coli are selected from the group consisting of DH10B, DH12S, XL-1Blue, XL-2Blue and DH5alpha.

In addition, the present invention relates to a method for the detection of contaminating microorganisms in a gene library consisting of microorganisms having a defect in at least one factor which is required to metabolize a compound or mixture of compounds comprising the steps of (a) growing said gene library on the growth medium of the invention; and (b) identifying said contaminating microorganism by visualizing its metabolic activity.
Growing the gene library on the growth medium of the invention may be effected according to conventional protocols, e.g. under sterile conditions and at 37°C. Means and methods to achieve identification as required in step (b) have been described herein above.

In a preferred embodiment of the method of the present invention said contaminating microorganisms lack said defect.

In a more preferred embodiment said microorganisms lacking said defect are bacteria.

In another more preferred embodiment of the method of the invention said microorganisms having said defect are bacteria.

Most preferably, said bacteria are E. coli.

In a further most preferred embodiment said E. coli have a defective lac-operon and lack the ability to metabolize saccharose, inosite and sodium thiosulfate.

In another further most preferred embodiment of the method of the invention said E. coli are selected from the group consisting of DH10B, DH12S, XL-1Blue, XL-2Blue and DH5alpha.

Further, most preferably said library spotted on a suitable carrier.

In a further most preferred embodiment spotting is effected in duplicate.
Spotting on duplicate carriers and subsequent analysis of the metabolic activity of microorganisms will enhance the reliability of the method of the invention. Thus, spotted microorganisms that display an enhanced metabolic activity as compared to library clones in identical positions of both carriers are confirmed to be contaminants.

The term "spotting" as used in accordance with the method of the present invention refers to a procedure, wherein an aliquot of a clone comprised by a gene library is transferred to a suitable carrier. Said carrier may be, for example, a microtitre plate, agar plate or membrane.

In particular, in cases where a large number of clones is to be spotted, said spotting onto a suitable carrier is advantageously effected or assisted by automation. More preferably, said automation is effected by an automated arraying, picking, spotting, pipetting or micropipetting device. Most preferably, said device is an arraying robot, picking robot, spotting robot, automated pipetting or micropipetting system. For example, a pipetting or micropipetting system may be advantageously applied to the transfer of microbial strains, e.g. bacterial strains. Other automation or robot systems that reliably transfer progeny of said microbial strains into predetermined arrays on said suitable carrier may also be employed. As the person skilled in the art will realize, the choice of said device will largely depend on the microbial strains used in said gene libraries.

In a further step, a contaminated clone may be visualized due to the metabolic activity of said contaminant. As has been explained above, contaminated clones in gene libraries can thus be identified without destroying the original clone.

Preferably, visualizing is assisted by visual means including digital image capture, digital image storage, digital image processing and/or digital image analysis.

Such visual means may incorporate a camera, for example a sensitive CCD camera that is suitable for luminescent and fluorescent detection, or may be colourimetric detection systems including computer-based scanners or colourimetric plate readers

Most preferably, said suitable carrier is an agar plate or an agar plate overlayed with a a neutral nylon- or nitrocellulose membrane or a microtiter plate, containing the growth medium of the present invention.

By "treated or overlaid with the growth medium of the invention" it is meant that depending on the nature of said suitable carrier, the growth medium of the invention may be applied by different well-known techniques. The growth medium may be applied during the production of said suitable carrier, e.g., for agar plates which comprise the growth medium of the invention, or it may be applied to membranes which may be impregnated by contacting them with agar plates which contain the growth medium of the invention. Of course, the growth medium of the invention may also conventionally be filled into microtiter plates or onto plastic plates.

In another most preferred embodiment of the method of the invention said carrier comprises a grid suitable for precise positioning of separate clones of the gene-library.

A suitable carrier in accordance with the present invention may also comprise a grid suitable for precise positioning of separate clones of the gene library. The "grid suitable for precise positioning" in accordance with the present invention refers to a device which allows the allocation of an aliquot of a clone of a gene library which has been spotted on to a carrier to said original clone in the gene library. Usually, said spotting procedure may be assisted by automation allowing a high reliability and quality control during the spotting procedure. Moreover, by using a grid suitable for precise positioning of the separate clones of the gene library the analysis and evaluation procedures may be also assisted by automation. Therefore, the whole procedure is very well-suited for high throughput screening.

Furthermore, most preferably also said spotting is assisted by automation.

In another most preferred embodiment of the method of the invention said visualizing of metabolic activity is monitored by a color change of a chromogenic substance.

In a further most preferred embodiment of the method of the invention said color change is effected by a pH-change.

In a furthermore most preferred embodiment of the method of the invention said chromogenic substance is Phenol Red.

Further most preferably, said chromogenic substance is ONPG.

In another most preferred embodiment of the method of the invention said visual analysis is based on the metabolism of sodium thiosulfate.

In a further most preferred embodiment of the method of the invention said metabolism of sodium thiosulfate is visualized by reaction with iron-III-citrate.

In a furthermore most preferred embodiment of the method of the invention said identification of said contaminating microorganism is registered by a computer assisted annotation program.

Since the method of the present invention may be performed assisted by automation the identification of a contaminant may be registered by a computer assisted annotation program which is advantageous for the administration and evaluation of data obtained with respect to microbial contamination of gene libraries. On the basis of said data, a computer assisted automation process may be provided which may perform further steps including elimination or purification steps of contaminated clones from said gene libraries. Thus, in addition, after evaluation of the data obtained by the method of the present invention said method may further comprise the steps of removing the contaminant from said suitable carrier and the step or recovering single uncontaminated clones from contaminated sets of unique gene libraries, thereby preventing the gene library from being destroyed by said contaminants and from contaminating other gene libraries which may be stored together with said gene library.

In accordance with the foregoing and most preferably, the method of the present invention further comprises the step of (ca) removing said contaminating microorganisms from said suitable carrier.

Further most preferably, said removal of the contaminants is assisted by automation.

Most preferably, the method of the present invention further comprises the step of (cb) recovering microorganisms having said defect from said growth medium as carrier.

Further most preferably, said recovery is assisted by automation.

In another further most preferred embodiment of the method of the invention said recovered microorganism is stroken out onto a suitable carrier as defined in any of the preceding claims wherein said carrier comprises or is contacted with the growth medium of the present invention.

A microorganism which has been recovered from a contaminated gene library may be stroken out onto a suitable carrier which may comprise or may be contacted with the growth medium of the present invention to again check the recovered microorganism for microbial contaminations.

Accordingly, in another further most preferred embodiment of the method of the inventions said method comprises re-isolating a pure colony from said carrier.

Finally, the present invention relates to a kit comprising (a) the growth medium of the present invention; (b) a suitable carrier as defined in the present invention; (c) optionally a spotting robot; and (d) optionally an optometric device for visual analysis.

The components of the kit of the invention may be packaged in containers such as buffers or cardboard containers. If appropriate one or more of said components may be packaged in one and the same container.

The figures show:
- Figure 1:: Figure 1 shows a membrane which has been placed on an Agar plate containing Contamination Detection Medium (CD Medium) and incubated for 24h. Clones from a gene library have been spotted in duplicate on the membrane. The grid of black ink spots allows identification and scoring of the spotted clones by a computer based annotation program. The yellow colonies represent a contaminated clone of said gene library spotted in duplicate.

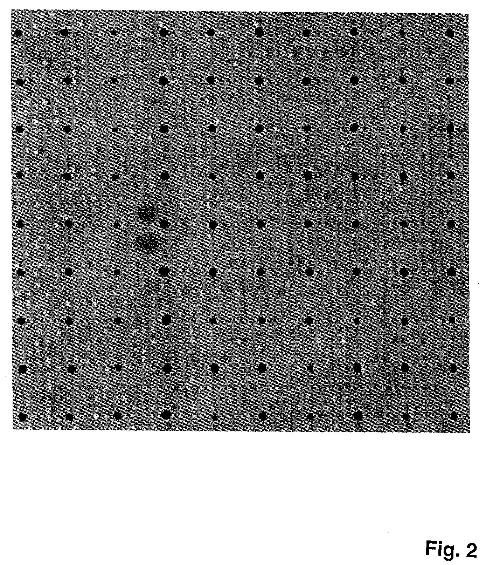

- Figure 2:: Figure 1 shows a membrane which has been placed on an Agar plate containing CD Medium and incubated for 24h. Clones from a gene library have been spotted in duplicate on the membrane. The grid of black ink spots allows identification and scoring of the spotted clones by a computer based annotative program. The red colonies represent a contaminated clone of said gene library spotted in duplicate.

The examples illustrate the invention.

### Example 1: Analysis of suitable components for a Contamination Detection Medium (CD Medium)

A new medium was designed based on the idea that metabolic activities present in most common forms of contamination, but not expressed in conventional E. coli strains of gene libraries should conveniently be detected. Five different E. coli strains commonly used for the production of gene libraries were used for this analysis, namely strains: DH10B, DH12S, XL1-Blue, XL2-Blue and DH5alpha. To identify suitable substrates, the ability of the test strains to metabolize 49 different sugars and 11 other substrates was tested (Table 1). The 28 substrates which are not metabolized by the test strains are indicted by bold letters. These substrates were used in an extensive database search to identify metabolic activities highly prevalent in potential contaminants. The analysis resulted in the identification of Lactose/ONPG, Saccharose, suitable substrates for the identification of metabolic activities of contaminants (Table 1*). In the CD-Medium /Table 2) these six substrates are dissolved in a suitable broth. In this example, metabolic activities of contaminants result either in a pH change, which is monitored with Phenol Red, or in the metabolism of the chromogenic substrate ONPG. The test may be done with clones growing on membranes supported on agar plates with CD-Medium or in liquid CD-Medium using microtitre plates.

**Table 1:**

| Analysis of substrate-metabolism of five E. coli strains commonly used in gene libraries. | | | | | |
|---|---|---|---|---|---|
| (+) ONPG is oNitrophenyl-β-D-Galacto-Pyranoside | | | | | |
| **E. coli strains substrates** | **DH10B** | **DH12S** | **XLIBlue** | **XLIIBlue** | **DH5alpha** |
| Glycerol | X | X | X | X | X |
| **Erythritol** | | | | | |
| **D-Arabinose** | | | | | |
| L-Arabinose | | | X | X | X |
| Ribose | X | X | X | X | X |
| D-Xylose | X | X | X | X | X |
| **L-Xylose** | | | | | |
| **Adonitol** | | | | | |
| **6 Methyl-xyloside** | | | | | |
| Galactose | | | X | X | X |
| D-Glucose | X | X | X | X | X |
| D-Fructose | X | X | X | X | X |
| D-Mannose | X | X | X | X | X |
| **L-Sorbose** | | | | | |
| Rhamnose | X | X | X | X | X |
| **Dulcitol** | | | | | |
| ***Inositol** | | | | | |
| Mannitol | X | X | X | X | X |
| Sorbitol | X | X | X | X | X |
| **Methyl-D-mannoside** | | | | | |
| **Methyl-D-glucoside** | | | | | |
| N Acetyl glucosamine | X | X | X | X | X |
| **Amygdaline** | | | | | |
| **Arbutine** | | | | | |
| **Esculine** | | | | | |
| Salicine | X | X | | | X |
| Cellobiose | X | | | | |
| Maltose | X | X | X | X | X |
| ***Lactose** | | | | | |
| Melibiose | X | X | X | X | X |
| ***Saccharose** | | | | | |
| Trehalose | X | X | X | X | X |
| **Inuline** | | | | | |
| **Melezitose** | | | | | |
| **D-Raffisnose** | | | | | |
| **Amidon** | | | | | |
| Glycogene | X | X | | | |
| Xylitol | X | | | | |
| Gentiobiose | | X | | | |
| **D-Turanose** | | | | | |
| **D-Lyxose** | | | | | |
| **D-Tagatose** | | | | | |
| **G-Fucose** | | | | | |
| L-Fucose | X | X | X | X | X |
| **D-Arabitiol** | | | | | |
| **L-Arabitiol** | | | | | |
| Gluconate | X | X | X | X | X |
| 2 ceto-gluconate | | | X | X | |
| 5-ceto-gluconate | X | X | X | X | X |
| ***ONPG+** | | | | | |
| **Arginine** | | | | | |
| Lysine | X | X | | | |
| **Ornithine** | | | | | |
| **Sodium Citrate** | | | | | |
| **Sodium Thiosulfate** | | | | | |
| **Urea** | | | | | |
| **Tryptophane** | | | | | |
| Tryptophane/indol | X | X | X | X | X |
| **Sodium Pyruvate** | | | | | |
| **Kehns Gelatine** | | | | | |

Legend to Table 1: The metabolic activities of E. coli strains DH10B, DH12S, XLIBlue, XLIIBlue and DH5alpha were tested. Substrates which are metabolized by the test strains are indicated (X). Substrates which are not metabolized by the test strains but by common contaminants are typed in bold letters.
Substrates which are finally included in the CD-Medium are indicated by an asterix (*).

### Example 2: Preparation of the CD Medium

For solution A, the following components were dissolved in a final volume of 500 ml H2O, the pH was adjusted to 7.0 with NaOH. The solution was autoclaved.

| Solution A: | | |
|---|---|---|
| Meat-extract | 6.0 | gram |
| Yeast-extract | 6.0 | gram |
| Peptone | 40.0 | gram |
| Sodium Chloride | 10.0 | gram |
| Agar (optional) | 15.0 | gram |

For solution B, the following components were dissolved in 500 ml H2O at 37° C and the solution was filtered sterile through a 0.2 um filter.

| Solution B: | | |
|---|---|---|
| Lactose | 20 | gram |
| Saccharose | 20 | gram |
| Inosite | 20 | gram |
| Iron III Citrate | 0.6 | gram |
| Na-Thiosulfate | 0.6 | gram |
| ONPG | 1.5 | gram |
| Phenol Red | 0.2 | gram |

For the most effective preparation of CD-Medium 500ml of Solution A was mixed with 500ml of Solution B.

### Example 3: Membranes showing E. coli clones grown on agar plates with CD-Medium

A membrane was placed on an agar plate containing CD-Medium. The clones of a gene library were spotted in duplicate onto the membrane and incubated over night. The grid of black ink spots allowed the scoring and identification of the clones with a computer based annotation program. The pale pink colonies were pure E. coli clones. The yellow (as shown in Figure 1) and red (as shown in Figure 2) pairs of colonies represented one contaminated clone each.

### Example 4: Application of the new CD-Medium to Gene Libraries

To be particularly practical for the analysis of large gene libraries which might contain more than 100 000 clones, it is necessary to adapt the test for contaminants to high throughput screening conditions. Agar plates with the size of 22x22 cm containing CD-Medium were covered with a neutral nitrocellulose or nylon membrane (like Optitran BAS 83 membrane from Schleicher & Schüll). 36 000 clones were spotted from microtiter plates onto the membrane with robotic spotting systems. With this method 180 000 clones can be screened with 1 liter CD-Medium.

After incubation of the membrane-agar plates, the E. coli clones grew up to small, pink colonies whereas even very low abundant contaminants were enhanced and formed yellow or even deep red colonies. A grid of black ink spots allowed the unambiguous positioning of the clones. The scoring and identification of the contaminated clones was registered by a computer assisted annotation program. Figure 1A shows a section of a membrane after over night incubation. Each clone was spotted twice to secure unambiguous identification. One clone spotted in duplicate showed a bright yellow color and could easily be recognized as a contaminant. The majority of contaminants had this appearance.

### References

1) Lennon et al. (1996), Genomics 33:151-152.
2) Kirby and Koller (1999), Abstract for the Human Genome Meeting 1999, Brisbane, Australia.

## Claims

1. A growth medium comprising a compound or a mixture of compounds, wherein said growth medium restricts growth of microorganisms having a defect in at least one factor which is required to metabolize said compound or mixture of compounds and wherein said growth medium promotes growth of microorganisms lacking said defect.

2. The growth medium of claim 1, wherein said microorganism lacking said defect are bacteria.

3. The growth medium of claim 1 or 2, wherein said microorganism having said defect is a bacterium.

4. The growth medium of claim 3, wherein said bacterium is E.coli.

5. The growth medium of claim 4, wherein said E.coli has a defective lac-operon and lacks the ability to metabolize saccharose, inosite and sodium thiosulfate.

6. The growth medium of claim 5, wherein said E.coli is selected from the group consisting of DH10B, DH125, XL-1Blue, XL-2Blue and DH5α.

7. The growth medium of any one of claims 1 to 6, wherein said compound is or said mixture of compounds comprises a carbohydrate.

8. The growth medium of claim 7, wherein said carbohydrate is selected from the group consisting of: erythritol, D-arabinose, L-xylose, adonitol, β-methylxyloside, L-sorbose, dulcitol, inositol, alpha-methyl-D-mannoside, alpha-methyl-D-glucoside, lactose, saccharose, inuline, melezitose, D-raffinose, D-turanose, D-lycose, D-tagatose, D-fucose, D-arabitol, L-arabitol, amygdaline, arbutin, esculine, amidon, and ONPG (=ortho-nitro-phenyl-galactoside).

9. The growth medium of any one of claims 1 to 8, wherein said compound is or said mixture of compounds comprises an amino acid or derivative thereof.

10. The growth medium of claim 9, wherein said amino acid or derivative thereof is selected from the group consisting of: arginine, ornithine, tryptophane, and urea.

11. The growth medium of any one of claims 1 to 10, wherein said compound is or said mixture comprises an inorganic compound.

12. The growth medium of claim 11, wherein said inorganic compound is selected from the group consisting of: sodium citrate, sodium thiosulfate, and sodium pyruvate.

13. The growth medium of any one of claims 1 to 12, wherein said compound is or said mixture of compounds comprises a complex substance.

14. The growth medium of claim 13, wherein said complex substance is Kohns gelatine.

15. The growth medium of any one of claims 1 to 14, wherein said mixture comprises at least saccharose, inosite, iron (III) citrate, sodium thiosulfate and ONPG.

16. The growth medium of claim 15, wherein said metabolization allows visual detection of microbial contaminants of gene libraries.

17. The growth medium of claim 16, wherein said visual analysis is based on pH-dependent color change of a chromogen.

18. The growth medium of claim 17, wherein said chromogen is Phenol Red.

19. The growth medium of claim 16, wherein said visual analysis is based on the metabolism of a chromogenic substrate.

20. The growth medium of claim 19, wherein said chromogenic substrate is ONPG.

21. The growth medium of claim 16, wherein said visual analysis is based on the metabolism of sodium thiosulfate.

22. The growth medium of claim 21, wherein said metabolism of sodium thiosulfate is visualized by reaction with iron-III-citrate.

23. The growth medium of any one of claims 1 to 22 further comprising Arbutin.

24. The growth medium of claim 1 or 23, wherein said microorganism lacking said defect is yeast.

25. The growth medium of claim 1, wherein said microorganism having said defect is yeast.

26. Use of the growth medium of any one of claims 1 to 25 for the detection of contaminating microorganisms in gene libraries.

27. The use of claim 25, wherein said contaminants are bacteria.

28. The use of claim 27, wherein said gene library is based on bacteria.

29. The use of claim 28, wherein said bacteria are E. coli.

30. The use of claim 29, wherein said E. coli comprise a defective lac-operon and lack the ability to metabolize saccharose, inosite and sodium thiosulfate.

31. The use of claim 29 or 30, wherein said E. coli are selected from the group consisting of DH10B, DH12S, XL-1Blue, XL-2Blue and DH5alpha.

32. A method for the detection of contaminating microorganisms in a gene library consisting of microorganisms having a defect in at least one factor which is required to metabolize a compound or mixture of compounds comprising the steps of
(a) growing said gene library on the growth medium of any one of claims 1 to 26; and
(b) identifying said contaminating microorganism by visualizing its metabolic activity.

33. The method of claim 32, wherein said contaminating microorganisms lack said defect.

34. The method of claim 33, wherein said microorganisms lacking said defect are bacteria.

35. The method of claim 33 or 34, wherein said microorganisms having said defect are bacteria.

36. The method of claim 35, wherein said bacteria are E. coli.

37. The method of claim 36, wherein said E. coli have a defective lac-operon and lack the ability to metabolize saccharose, inosite and sodium thiosulfate.

38. The method of claim 36 or 37, wherein said E. coli are selected from the group consisting of DH10B, DH12S, XL-1Blue, XL-2Blue and DH5alpha.

39. The method of any one of claims 32 to 38, wherein said library is spotted on a suitable carrier.

40. The method of claim 39 wherein said spotting is effected in duplicate.

41. The method of claim 39 or 40, wherein said suitable carrier is an agar plate or an agar overlayed with a neutral nylon- or nitrocellulose membrane or a microtiter plate, containing the growth medium of the present invention.

42. The method of any one of claims 39 to 41, wherein said carrier comprises a grid suitable for precise positioning of separate clones of the gene-library.

43. The method of any one of claims 39 to 42, wherein said spotting is assisted by automation.

44. The method of any one of claims 39 to 43, wherein said visualizing of metabolic activity is monitored by a color change of a chromogenic substance.

45. The method of claim 44, wherein said color change is effected by a pH-change.

46. The method of claim 44 or 45, wherein said chromogenic substance is Phenol Red.

47. The method of claim 44 or 45, wherein said chromogenic substance is ONPG.

48. The method of claim 44, wherein said visual analysis is based on the metabolism of sodium thiosulfate.

49. The method of claim 48, wherein said metabolism of sodium thiosulfate is visualized by reaction with iron-III-citrate.

50. The method of any one of claims 32 to 49, wherein said identification of said contaminating microorganism is registered by a computer assisted annotation program.

51. The method of any one of claims 38 to 50, further comprising the step of (ca) removing said contaminating microorganisms from said suitable carrier.

52. The method of claim 49, wherein said removal of the contaminants is assisted by automation.

53. The method of any one of claims 39 to 52, further comprising the step of (cb) recovering microorganisms having said defect from said growth medium as carrier.

54. The method of claim 53, wherein said recovery is assisted by automation.

55. The method of claim 53 or 54, wherein said recovered microorganism is stroken out onto a suitable carrier as defined in any of the preceding claims wherein said carrier comprises or is contacted with the growth medium of any one of claims 1 to 26.

56. The method of claim 54 comprising re-isolating a pure colony from said carrier.

57. A kit comprising
(a) the growth medium of any one of claims 1 to 26;
(b) a suitable carrier as defined in any of the preceding claims;
(c) optionally a spotting robot; and
(d) optionally an optometric device for visual analysis.
